**Europäisches Patentamt**

(19) **European Patent Office**

**Office européen des brevets**

(11) Publication number : **0 530 260 B1**

(12) # EUROPEAN PATENT SPECIFICATION

(45) Date of publication of patent specification :
22.06.94 Bulletin 94/25

(51) Int. Cl.[5] : **A23J 1/18, C12N 1/38**

(21) Application number : 91909773.3

(22) Date of filing : 21.05.91

(86) International application number :
PCT/EP91/00933

(87) International publication number :
WO 91/17669 28.11.91 Gazette 91/27

(54) PRODUCTION OF A PROTEINACEOUS COMPOSITION.

(30) Priority : 21.05.90 GB 9011347

(43) Date of publication of application :
10.03.93 Bulletin 93/10

(45) Publication of the grant of the patent :
22.06.94 Bulletin 94/25

(84) Designated Contracting States :
AT BE CH DE DK ES FR GB GR IT LI LU NL SE

(56) References cited :
FR-A- 2 244 407
US-A- 3 937 654

(73) Proprietor : ZENECA LIMITED
Imperial Chemical House, 9 Millbank
London SW1P 3JF (GB)

(72) Inventor : TRINCI, Anthony, Peter, Joseph
196 Bramhall Lane South
Bramhall, Stockport
Cheshire SK7 3AA (GB)
Inventor : ROBSON, Geoffrey, David 27 Athol
Road
Whalley Range
Manchester M16 8QW (GB)
Inventor : WIEBE, Marilyn, Gail 38 Heaton
Moor Road
Heaton Moor, Stockport
Cheshire SK4 4NX (GB)
Inventor : NAYLOR, Thomas, William Daphne
House 6 West Green
Heighington Village, Newton Aycliffe
Co. Durham DL5 6RA (GB)

(74) Representative : Locke, Timothy John et al
ICI Group Patents Services Dept.
PO Box 6
Shire Park
Bessemer Road
Welwyn Garden City Herts, AL7 1HD (GB)

## Description

This invention relates to a process for the production of a proteinaceous composition, particularly an edible proteinaceous composition.

GB-A-1210356, and GB-A-1346061 relate to the production of edible proteinaceous compositions by cultivating non-toxic strains of fungi under aerobic conditions in culture media containing a carbon source and other essential nutrients, cultivation being conducted in such a manner that the carbon source is the growth limiting nutrient.

GB-A-2137226 relates to an improvement in which cultivation is carried out in such a manner that oxygen is the growth limiting nutrient.

The products of the GB-A-1210356, GB-A-1346061 and GB-A-2137226 are intended to be meat analogues, e.g. beef or chicken analogues. To be successful for this purpose the proteinaceous products must have a texture, particularly a so-called "mouth feel" of the meat to be simulated. A difficulty in this respect is that the structure of the proteinaceous composition is dependent on the structure of the filamentous microorganisms of which it is composed, and that the structure of such filamentous microorganisms is dissimilar from that of the meat to be simulated. Variation in the texture of the meat analogue may be achieved by variation in the structure of the filamentous microorganism. It is important, therefore to be able to control the structure of the filamentous microorganism, in particular the extent of branching occurring in the hyphae. A useful indicator of the extent of branching is the "hyphal growth unit length". This may be determined by dividing the total length of all filaments of a mycelium by the total number of hyphal tips in the mycelium.

We have found that the branching of hyphae in a filamentous microorganism may be controlled through the regulation of the concentration of calcium ions in the medium in which the filamentous microorganism is cultivated.

Accordingly, the present invention provides a process for the production of a proteinaceous composition from a filamentous microorganism, wherein said filamentous microorganism has a hyphal growth unit length of a desired value, said process comprising:

i) cultivating said filamentous microorganism under aerobic conditions in a culture medium containing a carbon source and sources of other essential nutrients;

ii) testing for an indicator of hyphal branching for example the hyphal growth unit length ofsaid filamentous microorganism being cultivated in said medium; and

iii) controlling the calcium content of said medium to produce a result of the said test in a desired range.

The hyphal growth unit length can be measured directly for example by the use of a microsope but measurements of physical properties which are altered by changes in the hyphal growth unit length may be substituted for ease of measurement in a manufacturing process.

The invention also comprises a process for the production of a proteinaceous composition from a filamentous microorganism of the genus _Fusarium_ which process comprises cultivating _Fusarium_ under aerobic conditions in a culture medium containing a carbon source and sources of other essential nutrients characterised in that the culture medium comprises calcium ions in the range $10^{-9}$ to $10^{-5}$ molar and preferably $10^{-8}$ to $10^{-5}$ molar.

Cultivation may be conducted in a known manner such as in chemostat culture or turbidostat culture. In order that improved productivity may be obtained it is preferred, however, that the cultivation is conducted in turbidostat culture, i.e. in a culture wherein the majority of microbial cells present are not subjected to any specific nutrient limitation other than calcium limitation. When cultivation is conducted in turbidostat culture the growth of the microbial cells within the fermenter is controlled hydrodynamically.

Cultivation may be conducted batchwise, or continuously. Preferably, the cultivation is continuous.

The temperature at which the process is conducted may be selected according to the particular microorganism under cultivation so as to produce acceptable yields, and conversion ratios. Typical temperatures are within the range 25 to 34°C.

Similarly, the pH at which the process is conducted is preferably kept within a range at which maximum growth is exhibited for a particular microorganism. Typically the pH is within the range 5.0 and 8.0, and more usually about a pH of 6.

In the culture medium the carbon source may be any suitable carbon source, for example starch, starch containing materials, or products of their hydrolysis, e.g. glucose, sucrose, sucrose containing materials or hydrolysed sucrose, i.e. invert sugars, or mixtures thereof. Thus the carbon source may comprise hydrolysed potato, molasses, glucose, maltose, hydrolysed bean starch or cassava. Alternative carbon sources, such as carbon sources of animal origin e.g. milk whey, may also be used. Sufficient amounts of an assimilable carbon source together with other essential growth elements such as nitrogen, sulphur, phosphorus and trace elements are maintained in the culture medium so that growth of the microorganism is not limited by any of these nutrients. The dissolved oxygen tension (DOT) is maintained at a sufficient level and is preferably at a level at which the major proportion of the culture is not in oxygen limitation. In addition to the nutrients discussed above the presence of one or more vitamins such as biotin may be included in the culture medium.

During the process, culture medium is preferably continuously supplied to the culture and culture is continuously removed from the fermenter. The removed culture may then be treated to separate the filamentous microorganism therefrom. In a typical treatment the removed culture is heat treated, filtered and the resulting filter cake is then formulated into a state suitable for use in food products.

Where culture medium is continuously supplied to, and culture is continuously removed from the fermenter, the total number of individual mycelia in the fermenter is determined by the generation of new hyphal fragments (starter cells) through conidation and cell breakage. During cultivation it is important that starter cells are continuously produced in the culture. Ideally these starter cells should be generated at approximately the same rate at which cells are removed from the culture. If no starter cells are produced there will of course be a "wash-out" of the culture.

Generally, hyphal growth unit length is a direct function of the concentration of calcium ions within the fermenter. Usually, up to a calcium ion concentration of $10^{-5}$ M, the hyphal growth unit length varies proportionally to the logarithm of the calcium ion concentration. At calcium ion concentrations above about $10^{-5}$ M, hyphal growth unit length is less sensitive to variations in calcium ion concentration.

Calcium ion concentrations of about $10^{-9}$ M can be achieved with the use of chelating agents. In order to achieve such low concentrations of calcium ions most, if not all, of those raw materials, e.g. nutrients, not used as sources of calcium in the cultivation of the filamentous microorganism, are required to be substantially free from calcium.

Thus, cultivation of the filamentous microorganism may be conducted practically in a medium in which the calcium ion concentration is in the range $10^{-9}$ to $10^{-5}$ M. Preferably, the calcium ion concentration is between $10^{-8}$ and $10^{-5}$ M.

The time interval between the taking of successive samples, and the computation of the hyphal growth unit length of the filamentous microorganism therein, is determined by such factors as the size of the fermenter, the type of cultivation used, ie batch or continuous, chemostatic or turbidostatic, the specific filamentous microorganism employed etc. Usual time intervals between successive samples are in the range 4 hours to 24 hours.

Regulation of the calcium ion concentration as herein described may also affect the Specific Growth Rate, and the Growth Yield of the filamentous microorganism under cultivation. Specific Growth Rate is as defined by Steele et al, Journal of General Microbiology, 91, 362 - 368 (1975). Growth Yield is defined as the mass of biomass produced during cultivation per unit mass of carbon source supplied during cultivation, e.g. glucose.

Preferably the filamentous microorganism is a fungus, in particular a strain of <u>Fusarium</u>, and specifically <u>Fusarium graminearum</u> Schwabe strains IMI 145425 deposited 26 February 1970, and 154209 to 154213, deposited 14 January 1971, all of which became publicly available on 3 February 1976 or variants, or mutants of these strains. These specific strains of Fusarium graminearum are described in GB 1346061, the disclosure of which is incorporated therein by reference, and are deposited at the CAB International Mycological Institute, (IMI), Ferry Lane, Kew, Richmond, Surrey, England. Strain 145425 is also deposited under the Budapest Treaty as IMI 346762 and also ATCC 20334 at the American Type Culture Collection, 12301 Parklawn Drive, Rockville, Maryland 20852, USA.

The invention is illustrated by the following Examples. In these the microorganism <u>Fusarium graminearum</u> Schwabe strain IMI 145425 was used. This was maintained on soil at 4°C. Spores suspensions were harvested in distilled water from plate cultures grown for 7 days on Vogel's agar medium (H J Vogel Microbial Genetics Bulletin, 13, pp 42 to 44, 1956). The spores suspension was filtered through two layers of lens tissue (Whatman) to remove mycelial biomass and the spores were washed by centrifuging twice at 1500 g for 3 minutes, with resuspension in 10 ml of distilled water after each centrifugation.

<u>Fusarium graminearum</u> (IMI 145425) was grown in 20 ml volumes of Vogel's medium, containing glucose as the carbon source, lacking added calcium and containing 50 mM of 2-(N-Morpholino) ethanesulfonic acid (MES) as a buffer (pH 6) in 250 ml nephlos flasks.

Each flask was inoculated with 0.2 ml of a $10^{-6}$ spore suspension, and shaken at 200 rpm on a rotary shaker with a 2.5 cm stroke.

The basal calcium concentration of this medium was 3.5 $\mu$M (3.5 x $10^{-6}$ M) (measured using atomic absorption).

In the Examples, the hyphal growth unit length (G) was optically determined using mycelia suspended in growth medium and applied to microscope slides. For each value of hyphal growth unit length, as defined by

$$G = L/T,$$

where

L = total length of hyphae

T = number of hyphal tips,

25 mycelial fragments were measured along transects moving across the microscope slide. All fragments with five or more tips were measured unless they were entwined with other fragments to the extent of being inseparable.

## EXAMPLE 1

A series of experiments wherein the concentration of $Ca^{2+}$ ions was in the range $10^{-9}$ to $10^{-7}$ were conducted. Such concentrations were obtained by adding a filter sterilised (0.22 μm, Whatman) chelating agent, ethyleneglycol-bis (B-aminoethyl ether) $N,N,N^1,N^1$,-tetra-acetic acid (EGTA) to the medium, so as to achieve a chelating agent concentration of between 0.5 to 15 mM.

## EXAMPLE 2

A series of experiments similar to Example 1 were conducted in which the $Ca^{2+}$ ions were in the range $10^{-7}$ to $10^{-5}$. Such concentrations were obtained by adding various concentrations of filter sterilised calcium chloride ($CaCl_2$) to medium containing 1mM EGTA.

## EXAMPLE 3

A series of experiments similar to those in Examples 1 and 2 were conducted wherein the free $Ca^{2+}$ concentration was between $10^{-5}$ and $10^{-2}$ M. Such concentrations were obtained by the addition of calcium chloride alone to the medium.

Values for the Specific Growth Rate, Growth Yield in terms of conversion of nutrient carbon consumed to cell carbon produced, Hyphal Growth Unit Length, and Hyphal Diameter were determined for each series of experiments, and are displayed in Table I.

TABLE I

| Calcium Concentration (M) | Hyphal Growth Unit Length (μm) | Hyphal Diameter (μm) | Specific Growth Rate (μ) (hr$^{-1}$) | Growth Yield (Y) |
|---|---|---|---|---|
| $1.0 \times 10^{-2}$ | 355 | 2.2 | 0.24 | 0.38 |
| $5.0 \times 10^{-4}$ | 333 | 2.3 | 0.23 | 0.35 |
| $1.1 \times 10^{-6}$ | 286 | 2.2 | 0.22 | 0.32 |
| $1.4 \times 10^{-7}$ | 172 | 2.2 | 0.22 | 0.31 |
| $1.4 \times 10^{-8}$ | 130 | 3.4 | 0.21 | 0.29 |
| $4.8 \times 10^{-9}$ | 54 | 4.1 | 0.17 | 0.32 |

The products with hyphal growth unit lengths of less than 300 μm show good branching and are of attractive texture for certain food products.

## Claims

1. A process for the production of a proteinaceous composition from a filamentous microorganism said process comprising:

   i) cultivating said filamentous microorganism under aerobic conditions in a culture medium containing a carbon source and sources of other essential nutrients;

   ii) testing for an indicator of hyphal branching, for example the hyphal growth unit length of said filamentous microorganism; and

   iii) controlling the calcium content of said medium such that the result of the test is maintained in a desired range.

2.  A process for the production of a proteinaceous composition from a filamentous microorganism of the genus <u>Fusarium</u> which process comprises cultivating <u>Fusarium</u> under aerobic conditions in a culture medium containing a carbon source and sources of other essential nutrients characterised in that the culture medium comprises calcium ions in the range $10^{-9}$ to $10^{-5}$ molar and preferably $10^{-8}$ to $10^{-5}$ molar.

3.  A process as claimed in Claim 1 or 2 in which the cells present are not subjected to any nutrient limitation other than a limitation of calcium ions.

4.  A process as claimed in any preceding claim which is carried out continuously in which growth of the cells is controlled by controlling the dilution rate.

5.  A process as claimed in any preceding claim in which the cultivation is continuous.

6.  A process as claimed in any preceding claim in which the temperature is in the range 25°C - 34°C.

7.  A process as claimed in any preceding claim in which the pH is in the range 5.0 - 8.0.

8.  A process as claimed in any preceding claim in which the nutrient medium comprises as a carbon source starch, a product of hydrolysis of starch, glucose, sucrose or hydrolysed sucrose or mixtures thereof.

9.  A process as claimed in any preceding claim in which a chelating agent is present.

10. A process as claimed in any preceding claim in which the microorganism is at least one of <u>Fusarium</u> <u>graminearum</u> Schwabe strains IMI 145425 and 154209 to 154213 or a variant mutant or derivative thereof.

11. A process as claimed in any preceding claim in which culture removed from the process is heat treated, filtered and formulated into a state suitable for use in food products.


**Patentansprüche**

1.  Verfahren zur Herstellung einer proteinhaltigen Zusammensetzung aus einem filamentösen Mikroorganismus, wobei:
    (i) der filamentöse Mikroorganismus unter aeroben Bedingungen in einem Kulturmedium kultiviert wird, das eine Kohlenstoffquelle und Quellen für andere essentielle Nährstoffe enthält;
    (ii) ein Test auf einen Indikator der Hyphenverzweigung durchgeführt wird, zum Beispiel für die Einheitslänge des Hyphenwachstums des filamentösen Mikroorganismus; und
    (iii) der Calciumgehalt des Mediums gesteuert wird, so daß ein Testergebnis in einem gewünschten Bereich aufrechterhalten wird.

2.  Verfahren zur Herstellung einer proteinhaltigen-Zusammensetzung aus einem filamentösen Mikroorganismus der Gattung <u>Fusarium</u>, wobei <u>Fusarium</u> unter aeroben Bedingungen in einem Kulturmedium kultiviert wird, das eine Kohlenstoffquelle und Quellen für andere essentielle Nährstoffe enthält, und das Verfahren dadurch gekennzeichnet ist, daß das Kulturmedium Calciumionen in dem Bereich von $10^{-9}$ bis $10^{-5}$ molar und vorzugsweise von $10^{-8}$ bis $10^{-5}$ molar enthält.

3.  Verfahren nach Anspruch 1 oder 2, bei dem die vorliegenden Zellen keiner Nährstoffbeschränkung außer einer Beschränkung an Calciumionen unterliegen.

4.  Verfahren nach einem der vorangehenden Ansprüche, wobei das Verfahren kontinuierlich durchgeführt wird und bei dem das Wachstum der Zellen durch die Steuerung der Verdünnungsrate gesteuert wird.

5.  Verfahren nach einem der vorangehenden Ansprüche, bei dem die Kultivierung kontinuierlich erfolgt.

6.  Verfahren nach einem der vorangehenden Ansprüche, bei dem die Temperatur in dem Bereich von 25°C-34°C liegt.

7.  Verfahren nach einem der vorangehenden Ansprüche, bei dem der pH in dem Bereich von 5,0-8,0 liegt.

8.  Verfahren nach einem der vorangehenden Ansprüche, bei dem das Nährstoffmedium als Kohlenstoffquelle Stärke, ein Produkt der Hydrolyse von Stärke, Glukose, Saccharose oder hydrolysierter Saccharose oder deren Gemische aufweist.

9. Verfahren nach einem der vorangehenden Ansprüche, bei dem ein Komplexbildner verwendet wird.

10. Verfahren nach einem der vorangehenden Ansprüche, bei dem der Mikroorganismus wenigstens einer der Fusarium graminearum Schwabe Stämme IMI 145425 und 154209 bis 154213 oder eine Variantenmutante oder ein Abkömmling davon ist.

11. Verfahren nach einem der vorangehenden Ansprüche, bei dem die aus dem Verfahren entfernte Kultur hitzebehandelt, filtriert und in einen Zustand formuliert wird, der für die Verwendung in Nahrungsmittelerzeugnissen geeignet ist.

**Revendications**

1. Procédé de production d'une composition protéique à partir d'un micro-organisme filamenteux, lequel procédé comprend:
   (a) la culture aérobie du dit micro-organisme filamenteux dans un milieu contenant une source de carbone et des sources d'autres matières nutritives essentielles
   (b) un test indicateur de ramification hyphate, par exemple la longueur unitaire de croissance hyphale dudit micro-organisme filamenteux, et
   (c) le contrôle de la teneur en calcium du dit milieu, tel que le résultat du test se maintienne dans un intervalle désiré.

2. Procédé de production d'une composition protéique à partir d'un micro-organisme filamenteux du genre Fusarium, lequel procédé comprend la culture aérobie du Fusarium dans un milieu de culture contenant une source de carbone et d'autres sources de matières nutritives essentielles, caractérisé en ce que le milieu de culture contient des ions calcium à une concentration comprise entre $10^{-9}$ et $10^{-5}$ molaire et de préférence entre $10^{-8}$ et $10^{-5}$ molaire.

3. Procédé suivant la revendication 1 ou 2, dans lequel les cellules présentes ne sont soumises à aucune limitation en matière nutritive autre que celle en ions calcium.

4. Procédé suivant l'une quelconque des revendications précédentes, lequel est mené en continu et dans lequel la croissance des cellules est contrôlée par le contrôle du taux de dilution.

5. Procédé suivant l'une quelconque des revendications précédentes, dans lequel la culture est continue.

6. Procédé suivant l'une quelconque des revendications précédentes, dans lequel la température est comprise entre 25°C et 34°C.

7. Procédé suivant l'une quelconque des revendications précédentes, dans lequel le pH est compris entre 5,0 et 8,0.

8. Procédé suivant l'une quelconque des revendications précédentes, dans lequel le milieu nutritif contient comme source de carbone, de l'amidon, un produit d'hydrolyse de l'amidon, du glucose, du saccharose ou du saccharose hydrolysé ou leurs mélanges.

9. Procédé suivant l'une quelconque des revendications précédentes, dans lequel un agent de chélation est présent.

10. Procédé suivant l'une quelconque des revendications précédentes, dans lequel le micro-organisme provient d'au moins une des souches de Fusarium graminearum Schwabe IMI 145425 et 154209 à 154213 ou d'une variante mutant ou dérivé.

11. Procédé suivant l'une quelconque des revendications précédentes, dans lequel la culture obtenue par ce procédé est traitée à chaud, filtrée et formulée sous une forme appropriée à l'emploi dans les produits alimentaires.